# EUROPEAN PATENT APPLICATION

(11) **EP 4 471 748 A1**
(43) Date of publication of application: **04.12.2024**
(21) Application number: 23176689.0
(22) Date of filing: 01.06.2023
(51) Int. Cl.: G09B 23/30, G09B 23/34, G06T 19/20

(54) **METHOD OF PROVIDING MODIFIED MODEL DATA OF AN OBJECT**

(71) Applicant: Westfälische Wilhelms-Universität Münster, 48149 Münster (DE)
(72) Inventor: Hofbauer, Vincent Roman, 48149 Münster (DE); Gosheger, Georg, 48149 Münster (DE); Blaszczyk, Timo, 48147 Münster (DE)
(74) Representative: Schiweck Weinzierl Koch Patentanwälte Partnerschaft mbB

(57) **Abstract**

In various embodiments a method of providing modified model data of an object is provided, comprising: obtaining model data of the object; positioning at least one processing line through the object, wherein the processing line represents a trajectory of a tool to be introduced into the object; positioning a first target disk and a second target disk, spaced apart form one another, in the object, along the processing line; defining an exchange volume comprising the first target disk, the second target disk, a portion of a surface of the object surrounding the intersection of the processing line with the surface of the object, wherein the exchange volume is configured for attachment to the object; and obtaining the modified model data for the manufacture of a modified object, wherein the modified model data comprises model data for the exchange volume and model data for the remaining part of the object. Furthermore, a modified object, as manufactured by the method, is provided.

## Description

The present invention relates to a method of providing modified model data of an object, wherein the modified model data may be used to manufacture a modified object which can be used for training purposes and/or quality management relating to a processing step to be performed on the object. The present invention further relates to a modified object manufactured based on the model data provided by the method and comprising a main body and an exchange volume configured for attachment to the main body.

The evaluation of various processing or manufacturing steps, such as the provision of boreholes in a workpiece, both in medical testing series, but also in other industrial areas, has been cumbersome, costly and resource-intensive to date.

In order to achieve comparable testing series in medical drilling procedures and surgery simulations, the procedure is as follows. First, drilling into a commercial 3D model is performed. Then, a computer tomography of the 3D model, including an inserted wire or drill is performed. Next, the position of the inserted wire or drill is evaluated with the help of self-constructed graphical evaluation aids. Once the evaluation is concluded, the used 3D model is discarded, and a completely new 3D model is required for further test runs.

Manufacturers of medical devices (e.g. endoprostheses) currently use artificial bones (e.g. provided by Sawbones^{®} and Synbones^{®}) and human cadaveric specimens for learning new surgical methods. The correct execution and/or position of the inserted implants is visually checked or evaluated laboriously by means of 3D imaging (e.g. computer tomography), as outlined above.

According to the current state of the art, the evaluation of processing or manufacturing steps is costly and resource-intensive. In the medical field, new artificial bones or human specimens must be available for each individual application. In addition, a computer tomography is usually required for the evaluation of each test run or, instead, only a rough visual evaluation is performed. The current evaluation procedures are not only cost-intensive, but also time-consuming. In addition, computer tomography and digital evaluation by means of graphical evaluation aids increase the susceptibility to errors. This may lower accuracy of the evaluation.

In view of the problems outlined above, the present invention seeks to provide a fast and resource-saving alternative for the evaluation process of surgical and manufacturing steps in medical and industrial applications, respectively, without sacrificing qualitative evaluation.

In various embodiments, a method of providing modified model data of an object is provided. The model data may comprise data which describes a 3D structure of the object, e.g. in the form of an STL file or any other suitable file format. The object may be a any industrial workpiece on which a processing/manufacturing step by a tool or machine is to be performed. In the medical field, the object may be a bone structure, such as a shoulder blade or a portion of the hip socket.

In a first step, the method comprises obtaining model data of the object. This step may include loading the 3D model data comprised in a file into a corresponding program for computer-aided design (CAD). Overall, the method described herein may be a computer-implemented method. The 3D model data may have been originally obtained from any 3D imaging method, such as MRI, computer tomography or laser triangulation to name a few examples.

In a next step, the method comprises positioning at least one processing line through the object, wherein the processing line represents a trajectory of a tool to be introduced into the object. The trajectory may correspond to a target or reference trajectory which has been defined beforehand and is known to provide an optimal result if the tool (or subsequently an element) is introduced into the object along that trajectory in the corresponding processing step. The information about the target trajectory (e.g. two points or an initial point and a direction) may be loaded into the corresponding CAD program from a file. Thus, the processing line may be provided based on the information on the target trajectory stored in the file. Alternatively, the processing line may be positioned through the object by a user input in the CAD program.

In a next step, the method comprises positioning a first target disk and a second target disk, spaced apart form one another, in the object, along the processing line. Each of the target disks, which is displayed in the corresponding program in which the present method is executed, may extend beyond the surface of the object. Therefore, positioning the target disks in the object does not necessarily mean that the target disks are completely included in the object and not visible from the outside. Generally, the target disks may have the same or different dimensions. The target disks may be placed at their respective positions automatically, e.g. at a predefined distance from one another, based on the overall dimension of the object, or by corresponding user input into the CAD program. The processing line may be seen as a guide for the positioning of the at least two target disks. The step of adding the target disks may include positioning 3D representations of the target disks at desired locations of the object and fusing them with the model of the object.

In a next step the method comprises defining an exchange volume comprising the first target disk, the second target disk and a portion of a surface of the object surrounding the intersection of the processing line with the surface of the object, wherein the exchange volume is configured for attachment to the object. The exchange volume may further include volume portions of the object lying between those elements or so, for example, the volume of the object, which lies between the surface of the object where the tool is expected to penetrate the object and the target disk which is located farther away from that surface. Expressed differently, the exchange volume may include the stated elements and a portion of the volume of the object embedding those elements. Overall, the portion of the object which the processing line extends through up to the second target disk, i.e. the target disk lying farther away from the surface of the object where the tool is expected to penetrate the object, may be included in the exchange volume. The step of defining the exchange volume may comprise the step of cutting out a portion from the object in the CAD program which includes the target disks, a portion of the surface of the object lying "in front" of the first disk and the volume of the object lying between that portion of the surface and the second target disk. At least a portion of the outer shape of the exchange volume may thus correspond to the outer surface of the object.

In a next step, the method comprises obtaining the modified model data for the manufacture of a modified object, wherein the modified model data comprises model data for the exchange volume and model data for the remaining part of the object. The exchange volume and the remaining part of the object may be attached to one another and form the modified model. Thus, the remaining part of the object comprises an interface for the attachment of the exchange volume thereto. The interface may be a substantially flat surface to which the exchange volume is attached to or it may comprise a slot or an opening into which the exchange volume may be inserted. At the interface, elements for a secure and spatially stable fixation of the exchange volume to the remaining part of the object may be provided.

The modified model data may be used to manufacture the modified object, e.g. by means of an additive manufacturing technique such as 3D printing. The modified model may be then used, for example, to test a processing tool, such as a drill, which is operated automatically in a manufacturing process or handled manually by a surgeon in a training session. Furthermore, the processing environment in which the processing tool is used in combination with other systems, such as a 3D guiding system, may be tested.

The evaluation step, which follows the processing step (e.g. drilling) may include a direct comparison of the actual position of the intersection between the processing path (e.g. drilling path) and the respective target disk and the target position of the intersection between the processing line and that target disk, wherein the latter intersection defines the target intersection as planned in the CAD program. The comparison may be made directly on-site based on visual evaluation of the penetration point of the processing tool (e.g. drill) on the target disks. For example, when a drilling operation has been performed flawlessly, both actual penetration points coincide with the target penetration points on the two target disks. In case of a deviation, the extent thereof on the respective target disk can be assessed based on the distance between the actual penetration point and target penetration point. The obtained 3D trajectory of the actual drilling process can add to the improvement of the processing step, such as an implantation technique, and a learning effect can be verified by repeating the processing step. The processing step may be repeated by replacing the processed exchange volume by a "new" exchange volume and performing the processing step anew. It goes without saying that more than one processing line may be provided during the planning phase of the modified object, depending on the number of processing steps to be performed on the modified object. For example, if three drilling actions are to be performed, three processing lines may be provided, each of the processing lines intersecting at least one of the target disks and leading to a corresponding target penetration point. Thus, the present method may be used to manufacture a modified object on which complex multi-step manufacturing/processing methods may be tested and/or verified.

The testing/verification procedure based on the manufactured modified object may be advantageously used in the context of a surgical technique/implantation technique.

The method according to the present invention as well as the modified object which may be manufactured by means of the method can be applied in all fields of industry where a fast and accurate evaluation of precise placements of elements and drillings is necessary or offers process optimization. In the field of medicine and dentistry, new surgical methods, new intraoperative navigation systems or manual aiming devices can be tested by making use of the present method within a very short time or used to learn and improve surgical methods for implant positioning (orthopedics, trauma surgery, dentistry, mouth/jaw/facial surgery). This can be done in the context of workshops for operating room personnel and/or physicians as well as in the context of demonstrations e.g. at medical congresses and industry exhibitions.

According to further embodiments, the method may further include manufacturing, based on the modified model data, the exchange volume and the remaining part of the object to obtain the modified object. As mentioned before, an additive manufacturing technique may be used for that purpose. Multiple exchange volumes can be manufactured in accordance with the intended number of testing/verification runs to be performed on the modified object.

According to further embodiments of the method, the first target disk and the second target disk may be centered relative to the at least one processing line. Such an alignment of the at least two target disks may be advantageous, since a distance of the actual penetration point from the center of the respective target disk directly corresponds to a measure of the deviation. In order to facilitate the evaluation process, each target disk may comprise a distance indicator, e.g. in the form of concentric rings centered around the center of the target disk, the concentric rings being spaced from one another by a certain distance, such as 1 mm, 2 mm, 3 mm or more. At the same time, radial lines extending from the center of the target disk may be provided, spaced apart by a predefined angle from one another, such as 10°, 15°, 20°, 25° or more. The radial distance between the concentric rings and the angular shift between the radial lines may be chosen according to the requirements of the considered processing step to be tested. For example, these dimensions may be chosen based on the accepted tolerance in the underlying processing step.

According to further embodiments of the method, the first target disk and the second target disk may be positioned such that they are intersected by the at least one processing line at an angle of substantially 90°.

According to further embodiments of the method, the remaining part of the modified object may comprise an opening for insertion of the exchange volume. After performing the processing step on the modified object, the exchange volume may be removed from the opening to evaluate the target disks and replaced by a "fresh" exchange volume, which does not comprise any processing marks or traces. As noted previously, depending on the individual design of the modified object and on the size of the portion of the object where the target disks are implemented, at least a portion of at least one of the target disks may extend beyond the intrinsic surface of the object and thus be visible as a projection extending from the surface of the modified object.

According to further embodiments of the method, the exchange volume may have an asymmetric cross-section. This may be advantageous when the exchange volume is to be inserted into an opening provided in the remaining part of the modified object. An asymmetric cross section may stipulate a specific orientation of the exchange volume within the modified object and thus only allow correct insertion of the exchange volume into the remaining part of the modified object.

According to further embodiments of the method, the exchange volume may be configured for a press-fit connection with the modified object. A tight fit between the exchange volume and the remaining part of the modified object may be advantageous in that it reduces the positioning error between the intended positions of the target disks and their actual positions within the modified object.

According to further embodiments of the method, the exchange volume may include at least one first alignment element on its outer surface and the opening in the remaining part of the object may include at least one corresponding second alignment element on its inner surface, which is configured for engagement with the at least one first alignment element. The engagement of a pair comprising the first and second alignment elements may include the first alignment element sliding into the second alignment element, or vice versa. The alignment elements may be configured to couple pairwise, thus assuring - in addition to the asymmetric cross-section or as an alternative - that the exchange volume may be only attached to, e.g. inserted into, the modified object in a predetermined orientation. This may ensure precise evaluation of the accuracy of the processing step under test. In other words, the alignment elements may prevent incorrect insertion of the exchange volume into the modified object. Without the intent to limit the form and size of the alignment elements, the first element may comprise a protrusion with a triangular or rectangular cross-section extending longitudinally on the surface of the exchange volume and the second element may comprise a correspondingly shaped groove provided in the opening in the remaining part of the modified device, or vice versa.

According to further embodiments, the method may include splitting the exchange volume into at least two parts, which can be coupled to one another. Each of the parts may comprise one target disk or one of the parts may comprise two target disks. Expressed differently, the exchange volume may be designed or configured as a composite element including two parts which may be manufactured separately and then attached to one another (e.g. by a twist mechanism) to form the exchange volume. The splitting of the exchange volume into at least two parts may be performed in the CAD program which is used to design the modified object.

According to further embodiments of the method, which is based on the previous embodiment, the surface of each target disk may be visible from the outside on the corresponding part of the exchange volume. Such a configuration is advantageous in that the target disks may be readily inspected after separation of the two parts of the exchange volume. Such a configuration may be achieved by placing the target disks at the ends of the two parts, for example. The inner part of the exchange volume, i.e. the part which is located behind the outer part comprising a portion of the outer surface of the modified object which is subject to processing during the processing step, may comprise a target disk at each longitudinal end thereof or only at one longitudinal end thereof. Correspondingly, in the first case the outer part of the exchange volume does not comprise a target disk and in the second case the outer part comprises a target disk which may be advantageously arranged at its longitudinal end by which it is coupled to the inner part.

According to further embodiments of the method, the exchange volume may be formed as an integral part which comprises at least one slot for insertion of a target disk. That is, at least one target disk may be inserted into a slot or opening provided in the exchange volume. The other target disk may be attached to the longitudinal end of the exchange volume or it may be also insertable into a second slot or opening provided in the exchange volume. In any case, after removal of the exchange volume from the modified object, both target disks may be visually inspected.

According to further embodiments of the method, the object comprises a portion of a bone structure. The model data of the object may have been obtained from a medical imaging method (e.g. MRI, CT or others).

In further embodiments of the invention, a modified object is provided, including a main body and an exchange volume configured for attachment to the main body, wherein the exchange volume comprises a first target disk and a second target disk, spaced apart from one another. The main body may correspond to the remaining part of the modified object manufactured by the method of the present invention. Overall, the modified object may be one which has been manufactured by the method according to the invention described above.

According to further embodiments of the modified object, a portion of the outer surface of the at least a part of the exchange volume may form a surface of the main body. Generally, the exchange volume may be configured for attachment to or insertion into the remaining part of the modified object.

According to further embodiments of the modified object, the modified object is based on a bone structure. Expressed differently, the modified object may resemble a bone structure, such as a shoulder or a pelvis. Thus, the modified object may correspond to a modified bone structure, wherein the modification corresponds to the implementation of the exchange volume comprising at least two target disks into the modified object. As mentioned before, the target disks may or may not be visible from the outside of the modified object. Therefore, from its outer appearance, the modified bone structure may have the same outer appearance as the corresponding original object on the basis of which the modified bone structure has been designed and manufactured.

Further features of the modified object may be directly derived from aspects of the method of the present invention which have been described above.

The general description of the invention provided above and possible embodiments thereof, as well as its purposes and features, will become more apparent with reference to the following figures and detailed description, which are directed towards exemplary embodiments of the present invention.
Figure 1 illustrates an exemplary exchange volume.
Figure 2 illustrates an exemplary remaining part of the modified object.
Figure 3 shows a generic model containing an exemplary exchange volume and support frame.
Figure 4 shows a lateral view of exemplary model of a modified object in the form of a shoulder blade.
Figure 5 shows the exemplary model of a modified object from Figure 4 from a different perspective.

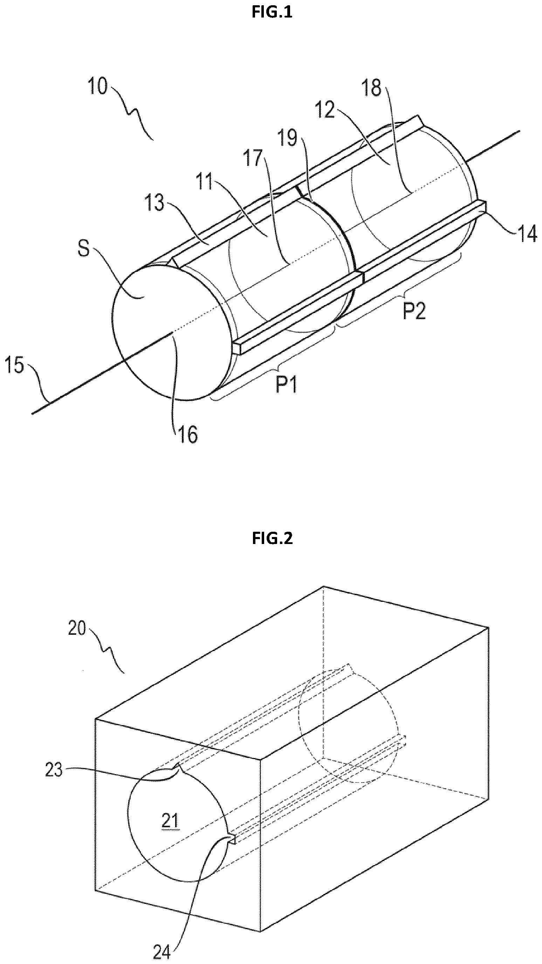

In Figure 1, an exemplary exchange volume 10 is shown, which may be manufactured based on the model data obtained from the method of providing modified model data of an object according to the invention. The exchange volume 10 has a substantially cylindrical shape and is configured for insertion into an opening provided in the remaining part of a corresponding modified object (shown in Figure 2). The exchange volume 10 includes a first part P1 and a second part P2 which may be separated from one another. A first target disk 11 is provided at the back surface of the first part P1 and a second target disk 12 is provided at the back surface of the second part P2. Alternatively, the first target disk 11 may be provided at the front surface of the second part P2, such that a target disk is attached to or provided at each end of the second part P2. Independent of the actual configuration, when the two parts P1, P2 are separated from one another, each target disk 11, 12 may be visually inspected to evaluate the precision of the performed processing step.

A processing line 15, which is used in the design phase of the modified object in the CAD program, is also shown. The processing line 15 penetrates the front surface S of the exchange volume 10 at a surface penetration point 16 and propagates into the exchange volume 10, penetrating the first target disk 11 at a first penetration point 17 and the second target disk at a second penetration point 18. In this example, the target disks 11, 12 are centered with respect to the processing line 15, such that the first and second penetration points 17, 18 correspond to the target penetration points. In the case of a modified object from the medical field, such as a prosthesis, the position of the processing line 15 is placed with reference to the surrounding structure represented by the remaining part of the modified object and based on experience and preoperative planning with the aim of providing an optimal surgical result.

On the surface of the exchange volume 10, a first alignment element 13 and a second alignment element 14 are provided. Each alignment element includes a protrusion extending longitudinally along the surface of the exchange volume 10, wherein the first alignment element 13 has a triangular cross-section and the second alignment element 14 comprises a rectangular cross-section.

In Figure 2, an exemplary remaining part of the modified object 20 (main body in the following) is shown, which is configured to receive the exchange volume 10 shown in Fig. 1. For this purpose, the main body 20 comprises an opening 21 which is configured for engagement with the exchange volume 10. In the sidewalls of the opening 21 a third alignment element 23 and a fourth alignment element 24 are provided. The third alignment element 23 has a negative form which corresponds to the form of the first alignment element 13 of the exchange volume 10 shown in Figure 1 and, correspondingly, the fourth alignment element 24 has a negative form which corresponds to the form of the second alignment element 14 of the exchange volume 10. Thus, there is only one orientation in which the exchange volume 10 may be inserted into the main body 20. Once the exchange volume 10 has been inserted into the main body 20, the modified object is obtained which may be used for testing and/or validation of a processing step, as already described in more detail above.

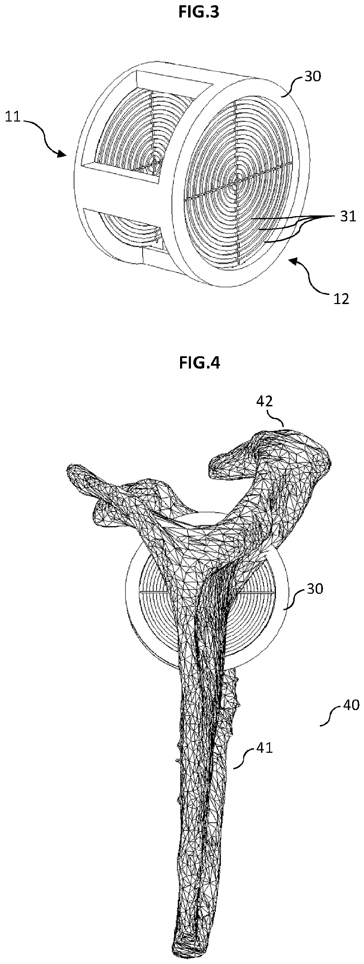

An exemplary generic model 30 comprising an exchange volume which may be used in a corresponding CAD program during the planning phase is shown in Fig. 3. The generic model 30 also includes a support frame which has a cylindrical shape and comprises a first target disk 11 and a second target disk 12 attached to each end. The support frame may be added automatically or manually to provide support for the two target disks 11, 12. The space between the target disks may be filled in order to mimic tissue or manufacturing of an object which needs to be processed through by a tool.

During the design phase, the generic model 30 of the exchange volume may be arranged such that the target disks 11, 12 are aligned along the processing line 15. In particular, the target disks 11, 12 may be centered relative to the processing line 15, as indicated in Figure 1. After positioning the generic model 30, it may be fused wit the model of the object, thus generating the modified object. Subsequently, the actual exchange volume 10 may be defined by cutting out a portion of the modified object which includes the generic model 30 with the two target disks 11, 12, a portion of the surface of the modified object and the volume of the modified object lying in between those elements. Additionally, as indicated in Figure 1, at least one alignment element may be added.

The target disks 11, 12 shown in Figure 3 comprise a structured surface which facilitates evaluation of the positions of the penetration points. In this exemplary case, the structure comprises an arrangement of concentric rings 31, which protrude from the otherwise flat surface of the target disks 11, 12 and which are spaced apart equidistantly. In addition, two lines running through the center of the target disks 11, 12 and intersecting at a right angle are provided to further facilitate the evaluation. If desired, more lines can be provided finer visual angular resolution. The arrangement of concentric rings 31 is provided on the back sides of the target disks 11, 12, i.e. the sides which face away from the processing surface of the corresponding object. The front sides of the target disks 11, 12 may be flat and not provided with any distance indicia. In general, the arrangement of concentric rings 31 may be provided on both sides of any of the target disks 11, 12 or only on one side.

Due to the structurally close interconnection of the support frame with the exchange volume, the support frame may be seen as a part of the exchange volume.

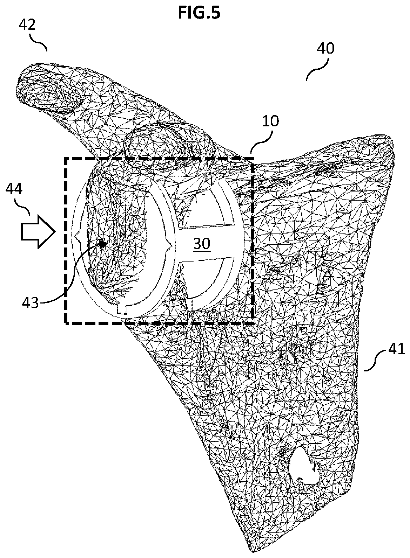

Figure 4 shows a lateral view of exemplary model of a modified object 40 in the form of a shoulder blade 41, including the acromion 42. The same modified object 40 is shown from a different perspective in Figure 5 which corresponds to a slightly elevated front view on the shoulder blade 41. In this view, the glenoid cavity 43 can be seen which, together with the humerus forms the glenohumeral joint.

As can be seen in Figures 4 and 5, the exchange volume 10 has been placed just behind the glenoid cavity 43 in order to allow testing of processing steps, such as drilling, into the glenoid cavity 43 coming from the direction indicated by the arrow 44. In this example, the structure of the generic model 30 is visible in the exchange volume 10. The exchange volume 10 is based on the generic model 30 and further includes a portion of the volume of the modified object 40 arranged within the generic model. The entire exchange volume 10 may be formed by using cube-shaped cut-out form and extracting a corresponding portion of the volume of the modified object 40 therefrom, as indicated by the dashed rectangle. Thus, the exchange volume 10 may have plane surfaces where the cube shaped cut-out form cuts through volume of the modified object 40. The remaining outer surface of the exchange volume 10 may correspond to the outer surface of the original object, i.e. the original shoulder structure without the exchange volume fused into it, or the surface of the generic model 30. The exchange volume 10 may attach seamlessly to the remaining part of the modified object 40. For example, the exchange volume 10 attached to the remaining part of the modified object 40, which includes a corresponding cut-out (not visible in Figures 4 and 5), by sliding it towards the shoulder blade 41 from the direction as indicated by the arrow 44 or perpendicular to it, i.e. from a direction pointing towards the plane of paper. Alignment elements for securing the exchange volume 10 in place may be provided at the interface between the exchange volume 10 and the remaining part of the modified object 40. In the shown example, the glenoid cavity 43 forms part of the exchange volume 10 and is thus replaced by a "fresh" and unscathed one such that the subsequent training or validation run will not be disturbed by processing traces thereon.

In the following, an exemplary evaluation process will be described for a test series in the context of medical drilling based on the modified object as shown in Figures 4 and 5. By making use of the method of the present invention and the corresponding modified object manufactured therewith the most important disadvantages of the current methods, as outlined in the background section of this description, may be overcome and overall the evaluation process may be significantly improved.

In a first step, a 3D model of the bone structure may be generated. The 3D model may include an STL 3D model on the basis of CT-slicing of a bone, for example.

In a next step, the processing line is placed in the model of the bone model.

In a next step, the two target disks, possibly supported by a generic frame, are placed along the processing line, preferably centered relative to the processing line, and fused with the 3D model of the bone.

In a next step, a 3D volume is defined which surrounds the target disks. Subsequently, the combination of those elements is sliced out, thus yielding the exchange volume. The exchange volume may comprise two parts, such that each of the target disks can be inspected after the exchange volume has been detached from the remaining part of the modified object.

In a next step, the parts of the modified object may be 3D printed, namely:
i) the bone model (remaining part of the modified object) with the interface for attachment of exchange volume comprising the target disks (needs to be printed only once); and
ii) the exchange volume comprising the target disks (multiple samples thereof can be manufactured in accordance with the intended number of trial runs)

In a next step, the exchange volume is attached to (e.g. inserted into) the remaining part of the modified object, thus giving rise to the modified object which is ready to use in the trial run. The attachment is done such that neither a rotation nor translation between the two entities is possible (key-lock-principle, provided by means of the alignment elements).

In a next step, the medical drilling training or trial run may be performed on the modified object to simulate a surgical procedure which is to be performed later on in the operation room. The drilling operation may be performed freehandedly or by means of conventional targeting devices.

In a next step, after the drilling procedure has been performed, the exchange volume comprising the target disks is removed and the two target disks are analyzed with respect to the positions of the penetration points, e.g. by visual inspection of the penetration points of the drill. For that purpose, the two parts of the exchange volume may be separated from one another (if exchange volume is constructed accordingly).

In a final step, the processing step is evaluated by a direct comparison of the actual penetration points on the target disks to the target penetration points. The details of an evaluation step, which follows the processing step, and possible conclusions which may be drawn therefrom have been already described in a previous section of this description.

## Claims

1. Method of providing modified model data of an object, comprising:
obtaining model data of the object;
positioning at least one processing line through the object, wherein the processing line represents a trajectory of a tool to be introduced into the object;
positioning a first target disk and a second target disk, spaced apart form one another, in the object, along the processing line;
defining an exchange volume comprising the first target disk, the second target disk and a portion of a surface of the object surrounding the intersection of the processing line with the surface of the object, wherein the exchange volume is configured for attachment to the object;
obtaining the modified model data for the manufacture of a modified object, wherein the modified model data comprises model data for the exchange volume and model data for the remaining part of the object.

2. Method of claim 1, further comprising:
manufacturing, based on the modified model data, the exchange volume and the remaining part of the object to obtain the modified object.

3. Method of claim 1 or 2,
wherein the first target disk and the second target disk are centered relative to the at least one processing line.

4. Method of any one of claims 1 to 3,
wherein the first target disk and the second target disk are positioned such that they are intersected by the at least one processing line at an angle of substantially 90°.

5. Method of any one of claims 1 to 4,
wherein the exchange volume has an asymmetric cross-section.

6. Method of any one f claims 1 to 5,
wherein the remaining part of the object comprises an opening for insertion of the exchange volume.

7. Method of claim 6,
wherein the exchange volume is configured for a press-fit connection with the modified object.

8. Method of claims 6 or 7,
wherein the exchange volume further comprises at least one first alignment element on its outer surface and the opening in the remaining part of the object comprises at least one corresponding second alignment element on its inner surface which is configured for engagement with the at least one first alignment element.

9. Method of any one of claims 1 to 8, further comprising:
splitting the exchange volume into at least two parts which can be coupled to one another, wherein preferably each of the parts comprises one target disk.

10. Method of claim 9,
wherein the surface of each target disk is visible from the outside on the corresponding part of the exchange volume.

11. Method of any one of claims 1 to 10,
wherein the exchange volume is an integral part which comprises at least one slot for insertion of a target disk.

12. Method of any one of the preceding claims,
wherein the object comprises a portion of a bone structure and the model data of the object has been preferably obtained from a medical imaging method.

13. Modified object, comprising:
a main body; and
an exchange volume configured for attachment to the main body, wherein the exchange volume comprises a first target disk and a second target disk, spaced apart form one another.

14. Modified object of claim 13,
wherein a portion of the outer surface of the at least a part of the exchange volume forms a surface of the main body.

15. Modified object of claim 13 or 14, wherein,
the modified object is based on a bone structure.
